Europäisches Patentamt

(19) European Patent Office

Office européen des brevets

(11) Publication number: **0 078 599**
**B1**

(12) **EUROPEAN PATENT SPECIFICATION**

(45) Date of publication of patent specification: **12.11.86**

(51) Int. Cl.⁴: **C 08 B 37/16**

(21) Application number: **82304535.6**

(22) Date of filing: **27.08.82**

(54) Complex compounds based on cyclodextrins.

(30) Priority: **01.09.81 JP 138048/81**

(43) Date of publication of application:
**11.05.83 Bulletin 83/19**

(45) Publication of the grant of the patent:
**12.11.86 Bulletin 86/46**

(84) Designated Contracting States:
**AT BE CH DE FR GB IT LI NL SE**

(56) References cited:
**DE-A-3 035 086**

**CHEMICAL ABSTRACTS, vol. 90, 1979, page
305, no. 43740s, Columbus, Ohio, US T. NAGAI
et al.: "Bioavailability of powdered inclusion
compounds of nonsteroidal antiinflammatory
drugs with beta-cyclodextrin"**

The file contains technical information
submitted after the application was filed and
not included in this specification

(73) Proprietor: **TEIKOKU CHEMICAL INDUSTRY
CO., LTD.
1-18, 1-chome Kitahorie
Nishi-ku Osaka (JP)**

(72) Inventor: **Shinoda, Masamitu
336-2, Nyoidani
Minou-shi Osaka-fu (JP)**
Inventor: **Tanaka, Ikuo
1352, Aomadani
Minou-shi Osaka-fu (JP)**
Inventor: **Yasuda, Tadahiko
34-1, Fukakusa Ganjo-cho
Fushimi-ku Kyoto-shi (JP)**
Inventor: **Nakajima, Isao
4-4-17, Hattorihonmachi
Toyonaka-shi Osaka-fu (JP)**
Inventor: **Adachi, Tutomu
7-3,2-chome, Nishidai
Itami-shi Hyogo-ken (JP)**
Inventor: **Ikeda, Giichi
91, Minamitukiyomi-cho Nishinanajo
Shimogyo-ku Kyoto-shi (JP)**

(74) Representative: **Perry, Robert Edward et al
GILL JENNINGS & EVERY 53-64 Chancery Lane
London WC2A 1HN (GB)**

Courier Press, Leamington Spa, England.

0 078 599

(58) References cited:

THE JOURNAL OF THE AMERICAN CHEMICAL
SOCIETY, vol. 103, no. 7, April 8, 1981, pages
1750-1757 R.I. GELB et al.: "Binding
mechanisms in cyclohexaamylose complexes"

CHEMICAL ABSTRACTS, vol. 90, 1979, page
311, no. 43812s, Columbus, Ohio, US
DIE STÄRKE, vol. 30, no. 12, December 1978,
pages 427-431 J. SZEJTLI: "Neue
Untersuchungsmethoden in der
Cyclodextrinchemie"

**Description**

This invention relates to novel complex compounds.

The acid addition salts of (2' - benzyloxycarbonyl)phenyl - *trans* - 4 - guanidinomethylcyclohexanecarboxylate ("GCP" herein) can inhibit proteolytic enzymes such as trypsin, chymotrypsin, thrombin, kallikrein and urokinase. These compounds are therefore of great pharmaceutical potential, but have hitherto had to be administered in very large amounts, to obtain a desired effect, owing to their limited absorption. This is a considerable problem, having regard to the potential effect of the given compounds in ulcer treatment.

Complex compounds according to the present invention are of GCP acid addition salts with a cyclodextrin. A pharmaceutical composition according to the invention comprises such a complex compound and a physiologically acceptable excipient.

Examples of suitable acid addition salts are GCP hydrochloride, hydrobromide, hydroiodide, carbonate, acetate, sulfate, p-toluenesulfonate, methanesulfonate, lactate, maleate, fumarate, tartrate and citrate. The cyclodextrin may be, for example, α-cyclodextrin, γ-cyclodextrin or, preferably, β-cyclodextrin. The complex compound generally comprises from 0.1 to 3.0 mol cyclodextrin per mol GCP acid addition salt.

The complex compound of the invention may be simply prepared by reacting a GCP acid addition salt with a cyclodextrin. By way of example, the reactants may be dissolved in water, with heating if desired; the solution is then subjected to conventional freeze-drying or concentration, to separate the product.

The following Examples illustrate how the complex compounds of the invention may be prepared.

Example 1

46.3 g (0.1 mole) of (2' - benzyloxycarbonyl)phenyl - *trans* - 4 - guanidinomethylcyclohexanecarboxylate hydrochloride monohydrate and 113.5 g (0.1 mole) of β-cyclodextrin were added to 600 ml water. The mixture was heated to 70—75°C to obtain a solution which was subjected to freeze-drying to obtain 155 g of a complex of (2' - benzyloxycarbonyl)phenyl - *trans* - 4 - guanidinomethylcyclohexane-carboxylate hydrochloride with β-cyclodextrin.

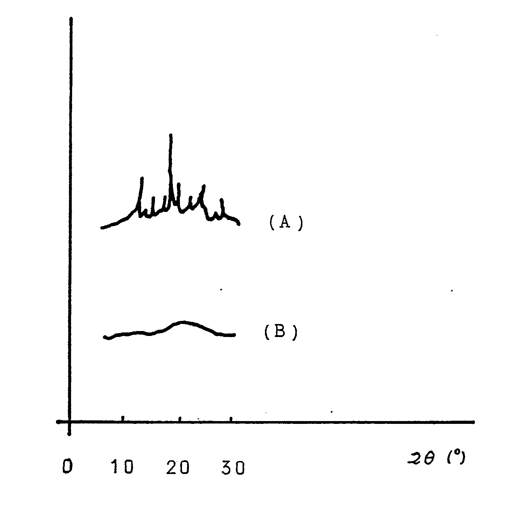

In the accompanying drawings, Figure 1 shows the powder X-ray diffraction patterns for GCP HCl (A) and for the product of Example 1 (B). It will be apparent that these patterns are quite different; the novel complex is apparently of amorphous structure.

By way of further comparison, GCP HCl and the complex of Example 1 were analysed by thin layer chromatography. On development with acetonitrile, their respective Rf values were 0.9 and 0.0.

Example 2

10 g of (2' - benzyloxycarbonyl)phenyl - *trans* - 4 - guanidinomethylcyclohexanecarboxylate hydrochloride and 14.1 g of β-cyclodextrin were suspended in 150 ml of water and heated. At 75°C, a clear solution was obtained; this solution, after having been maintained at the same temperature for 30 minutes, was concentrated under reduced pressure to obtain a solid product. After washing with acetonitrile and drying, 14.4 g of complex product were obtained.

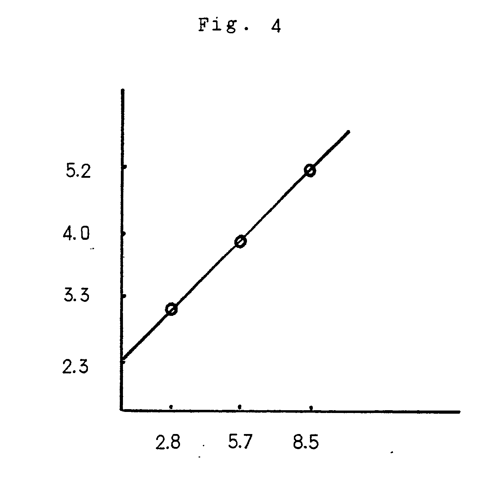

Figure 4 is a phase solubility diagram (in water, at 25°C), wherein the ordinate shows the amount of GCP HCl (mg/ml) and the transverse axis shows the amount of β-cyclodextrin (mg/ml).

For pharmaceutical use, e.g. in the treatment of ulcers, a complex of the invention may be administered orally or parenterally. For such purposes, the complex may be compounded in the form of tablets, capsules, granules or fine powders, for oral administration, or as injectable fluids or suppositories for parenteral administration.

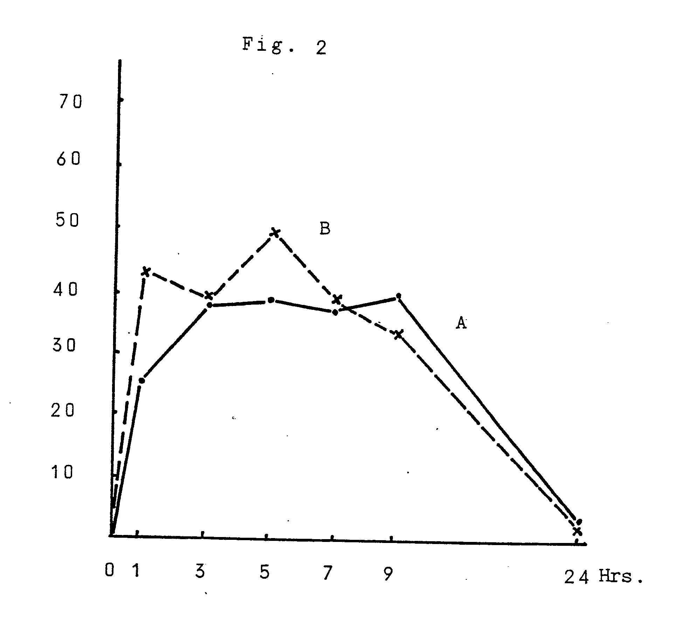

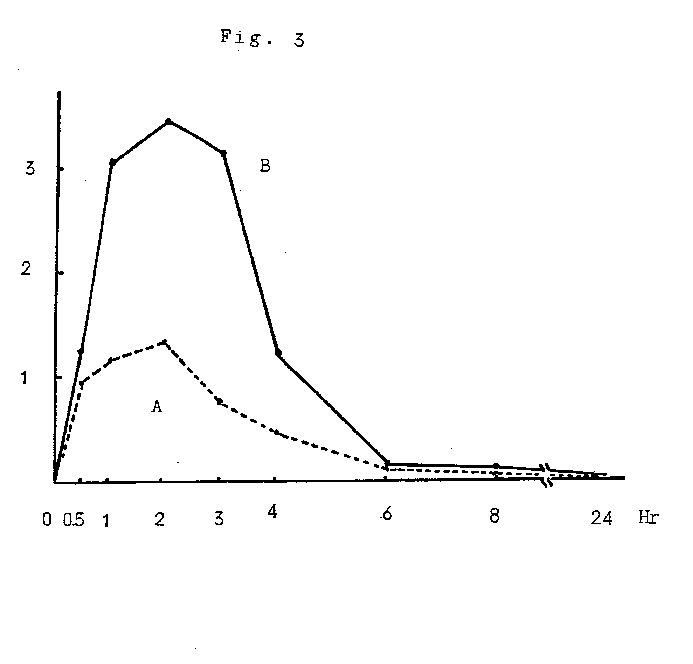

In order to illustrate the pharmaceutical utility of the novel complexes, GCP HCl (A) was compared with the product of Example 1 (B). In the accompanying drawings, Figure 2 shows the variation of salicylic acid concentration in blood plasma for GCP HCl (A) and for complex (B), each administered in an amount of 100 mg (as GCP HCl)/kg, with respect to time. In another test, 14 C-labelled compounds were administered orally to a group of rats (dose: 100 mg/kg, including 219 μCi labelled compound), and the variation of GCP HCl eq. in blood with time was determined. Figure 3 shows the results, in terms of GCP HCl eq. (μg/ml) with respect to time (hour).

Pharmacological test results are shown below:

In each test, a group of rats was used and the averaged test results are shown. For each dosage administered, the GCP HCl equivalent is shown in brackets.

a) Inhibition of stress ulcer (water-dipping method):

| | dosage (mg/kg) | coefficient of ulceration | inhibition (%) |
|---|---|---|---|
| control | | 25.8±2.9 | |
| complex | 320.5 | 23.3±2.4 | 9.7 |
| | 641.0 (200) | 15.4±3.1 | 40.3 |
| | 1282.1 (400) | 11.2±2.3 | 56.5 |
| | 2564.1 (800) | 7.6±1.3 | 70.5 |

The complex was administered orally 10 minutes before inducing stress.
b) Inhibition of indomethacin-induced ulcer:

| | dosage (mg/kg) | coefficient of ulceration | inhibition (%) |
|---|---|---|---|
| control | | 23.3±3.4 | |
| complex | 120.2 (37.5) | 11.6±3.5 | 50.2 |
| | 240.4 (75) | 5.6±1.1 | 76.0 |

The complex was administered orally 10 minutes before the administration of indomethacin (20 mg/kg).
c) Effect on secretion of gastric juice:

| | dosage (mg/kg) | amount of gastric juice (ml) | inhibition (%) | acid amount (µEq/hr) | inhibition (%) |
|---|---|---|---|---|---|
| control | | 7.0±0.6 | | 94.6±13.2 | |
| complex | 240.4 (75) | 5.3±0.7 | 24.3 | 70.6±11.2 | 25.4 |
| | 961.5 (300) | 5.0±0.4 | 28.6 | 67.4±8.4 | 28.8 |

The complex was administered into the duodenum immediately after tying the pylorus, and gastric juice was collected 7 hours later.

**Claims for the Contracting States: BE, CH, DE, FR, GB, IT, LI, LU, NL, SE**

1. A complex compound of an acid addition salt of (2' - benzyloxycarbonyl)phenyl - *trans* - 4 - guanidinomethylcyclohexanecarboxylate with a cyclodextrin.

2. A complex according to claim 1, wherein the acid addition salt is selected from the hydrochloride, hydrobromide, hydroiodide, carbonate, acetate, sulfate, p-toluenesulfonate, methanesulfonate, lactate, maleate, fumarate, tartrate and citrate.

3. A complex according to claim 1 or claim 2, wherein the cyclodextrin is selected from α-cyclodextrin, β-cyclodextrin and γ-cyclodextrin.

4. The complex of (2' - benzyloxycarbonyl)phenyl - *trans* - 4 - guanidinomethylcyclohexanecarboxylate hydrochloride and β-cyclodextrin.

5. A pharmaceutical composition comprising a complex according to any preceding claim and a physiologically acceptable excipient.

6. A complex according to any of claims 1 to 4, or a composition according to claim 5, for use in the treatment of ulcers.

**Claims for the Contracting State: AT**

1. A process for preparing a complex compound of an acid addition salt of (2' - benzyloxycarbonyl)phenyl - *trans* - 4 - guanidinomethylcyclohexanecarboxylate with a cyclodextrin, which comprises reacting the acid addition salt with the cyclodextrin.

2. A process according to claim 1, wherein the acid addition salt is selected from the hydrochloride, hydrobromide, hydroiodide, carbonate, acetate, sulfate, p-toluenesulfonate, methanesulfonate, lactate, maleate, fumarate, tartrate and citrate.

3. A process according to claim 1 or claim 2, wherein the cyclodextrin is selected from α-cyclodextrin, β-cyclodextrin and γ-cyclodextrin.

4. A process according to claim 1, which comprises reacting (2' - benzyloxycarbonyl)phenyl - *trans* - 4 - guanidinomethylcyclohexanecarboxylate hydrochloride and β-cyclodextrin.

**Patentansprüche für die Vertragsstaaten: BE, CH, DE, FR, GB, IT, LI, NL, SE**

1. Komplexverbindung aus einem Säureadditionssalz von (2' - Benzyloxycarbonyl)phenyl - trans - 4 - guanidinomethylcyclohexancarboxylat mit einem Cyclodextrin.

2. Komplex nach Anspruch 1, worin das Säureadditionssalz unter dem Hydrochlorid, Hydrobromid, Hydrojodid, Carbonat, Acetat, Sulfat, p-Toluolsulfonat, Methansulfonat, Lactat, Maleat, Fumarat, Tartrat und Citrat ausgewählt ist.

3. Komplexverbindung nach Anspruch 1 oder Anspruch 2, worin das Cyclodextrin unter α-Cyclodextrin, β-Cyclodextrin und γ-Cyclodextrin ausgewählt ist.

4. Der Komplex aus (2' - Benzyloxycarbonyl)phenyl - trans - 4 - guanidinomethylcyclohexancarboxylat - hydrochlorid und β-Cyclodextrin.

5. Pharmazeutische Zusammensetzung, umfassend einen Komplex gemäß einem der vorstehenden Ansprüche und ein physiologisch annehmbares Excipiens.

6. Komplex nach einem der Ansprüche 1 bis 4, oder Zusammensetzung nach Anspruch 5, zur Anwendung in der Behandlung von Geschwüren.

**Patentansprüche für den Vertragsstaat: AT**

1. Verfahren zur Herstellung einer Komplexverbindung aus einem Säureadditionssalz von (2' - Benzyloxycarbonyl)phenyl - trans - 4 - guanidinomethylcyclohexancarboxylat mit einem Cyclodextrin, welches ein Umsetzen des Säureadditionssalzes mit dem Cyclodextrin umfaßt.

2. Verfahren nach Anspruch 1, worin das Säureadditionssalz unter dem Hydrochlorid, Hydrobromid, Hydrojodid, Carbonat, Acetat, Sulfat, p-Toluolsulfonat, Methansulfonat, Lactat, Maleat, Fumarat, Tartrat und Citrat ausgewählt wird.

3. Verfahren nach Anspruch 1 oder Anspruch 2, worin das Cyclodextrin unter α-Cyclodextrin, β-Cyclodextrin und γ-Cyclodextrin ausgewählt wird.

4. Verfahren nach Anspruch 1, welches ein Umsetzen von (2' - Benzyloxycarbonyl)phenyl - trans - 4 - guanidinomethylcyclohexancarboxylat - hydrochlorid mit β-Cyclodextrin umfaßt.

**Revendications pour les Etats Contractants: BE, CH, DE, FR, GB, IT, LI, NL, SE**

1. Un complexe d'un sel du trans - 4 - guanidinométhylcyclohexane - carboxylate de (2' - benzyloxycarbonyl)phényle formé par addition avec un acide et d'une cyclodextrine.

2. Un complexe selon la revendication 1, dans lequel le sel formé par addition avec un acide est choisi parmi le chlorhydrate, le bromhydrate, l'iodhydrate, le carbonate, l'acétate, le sulfate, le p-toluènesulfonate, le méthansulfonate, le lactate, le maléate, le fumarate, le tartrate et le citrate.

3. Un complexe selon la revendication 1 ou 2, dans lequel la cyclodextrine est choisie parmi l'α-cyclodextrine, la β-cyclodextrine et la γ-cyclodextrine.

4. Le complexe du chlorhydrate du trans - 4 - guanidinométhylcyclohexane - carbonate de (2' - benzyloxycarbonyl)phényle et de la β-cyclodextrine.

5. Une composition pharmaceutique comprenant un complexe selon l'une quelconque des revendications qui précèdent et un excipient acceptable pour l'usage pharmaceutique.

6. Un complexe selon l'une quelconque des revendications 1 à 4, ou une composition selon la revendication 5, pour l'utilisation dans le traitement des ulcères.

**Revendications pour l'Etat Contractant: AT**

1. Un procédé pour préparer un complexe d'un sel du trans - 4 - guanidinométhylcyclohexane - carboxylate de (2' - benzyloxycarbonyl)phényle formé par addition avec un acide et d'une cyclodextrine, procédé qui consiste à faire réagir le sel formé par addition avec un acide avec la cyclodextrine.

2. Un procédé selon la revendication 1, dans lequel le sels formé par addition avec un acide est choisi parmi le chlorhydrate, le bromhydrate, l'iodhydrate, le carbonate, l'acétate, le sulfate, le p-toluènesulfonate, le méthanesulfonate, le lactate, le maléate, le fumarate, le tartrate et le citrate.

3. Un procédé selon la revendication 1 ou 2, dans lequel la cyclodextrine est choisie parmi l'α-cyclodextrine, la β-cyclodextrine et la γ-cyclodextrine.

4. Un procédé selon la revendication 1, qui consiste à faire réagir le chlorhydrate du trans - 4 - guanidinométhylcyclohexane - carboxylate de (2′ - benzyloxycarbonyl)phényle avec la β-cyclodextrine.

(A)

(B)

0    10    20    30                    $2\theta$ (°)

Fig. 2

Fig. 3

Fig. 4